# EUROPEAN PATENT APPLICATION

(11) **EP 4 393 521 A1**
(43) Date of publication of application: **03.07.2024**
(21) Application number: 22866196.3
(22) Date of filing: 20.06.2022
(51) Int. Cl.: A61L 27/24, A61L 27/50, A61L 27/52, C07K 14/00

(54) **BIONIC CORNEA AND PREPARATION METHOD THEREFOR**

(30) Priority: 13.09.2021 CN 202111059840
(71) Applicant: Eosvision Medtech Technology Co., Ltd., Suzhou, Jiangsu 215500 (CN)
(72) Inventor: WANG, Chongyu, Suzhou, Jiangsu 215500 (CN); ZHANG, Jiahui, Suzhou, Jiangsu 215500 (CN); ZHANG, Jun, Suzhou, Jiangsu 215500 (CN); LIU, Murong, Suzhou, Jiangsu 215500 (CN); HE, Chaoxian, Suzhou, Jiangsu 215500 (CN)
(74) Representative: Potter Clarkson
(86) International application number: PCT/CN2022/099756
(87) International publication number: WO 2023/035721

(57) **Abstract**

The present application relates to the field of biotechnology, and relates to a bionic cornea and a preparation method therefor. The present application provides a bionic cornea. A raw material of the bionic cornea is pegylated collagen. The pegylated collagen comprises collagen and PEG-40k and PEG-20k which are modified on the collagen. Modifications of PEG-40k and PEG-20k can significantly improve the cross-linking performance of the collagen, so that the strength of the cornea of the present application is increased by nearly 20 times compared with the strength of the cornea prepared from collagen, and is increased by nearly 12 times compared with the strength of the cornea prepared from the existing another pegylated collagen. Therefore, the cornea of the present application has good application prospect in the field of corneal transplantation. A molar ratio of PEG-40k to PEG-20k is 2-3:1. The effect of improving the cross-linking performance of the collagen by PEG modification at this molar ratio is better, and the strength of the cornea of the present application can be further improved.

## Description

### CROSS-REFERENCES TO RELATED APPLICATION

This application claims the priority of Chinese patent application submitted to the China National Intellectual Property Administration of the People's Republic of China on September 13, 2021, with the application number 202111059840.1 and the invention title "BIONIC CORNEA AND PREPARATION METHOD THEREFOR", all of which are incorporated herein by reference.

### FIELD OF THE INVENTION

The present application relates to a method for preparing bionic cornea and its application, and belongs to the field of biotechnology.

### BACKGROUND OF THE INVENTION

The cornea is the convex, highly transparent substance at the front of the eye. It is transversely oval in shape and covers the iris, pupil and anterior chamber, and provides most of the refractive power of the eye. Coupled with the refractive power of the lens, light can be accurately focused on the retina to form an image. The cornea has very sensitive nerve endings. If a foreign object comes into contact with the cornea, the eyelids will involuntarily close to protect the eyes. In order to maintain transparency, the cornea has no blood vessels and obtains nutrients and oxygen through tears and aqueous humor.

The cornea is very fragile. Eye trauma, inflammation, allergic reactions, physical damage, chemical burns, strenuous exercise, overuse of eyes, etc. can all lead to corneal pathology. Once the cornea becomes diseased, it will lead to obvious eye symptoms, such as eye pain, photophobia, tearing, decreased visionvision loss, etc., and in severe cases, blindness.

Corneal transplantation is a treatment method that replaces a the patient's existing diseased cornea with a normal cornea to restore vision in the affected eye or control corneal disease, improve vision, or treat certain corneal diseases. Some corneal diseases that cause severe visual impairment or even blindness in patients can be completely treated through corneal transplantation, helping these unfortunate patients stay away from pain. Because the cornea itself does not contain blood vessels and is in an "immune pardon" status, the success rate of corneal transplantation ranks first among other allogeneic organ transplants.

However, corneal resources are limited and far from meeting the needs of patients. To solve this problem, some researchers proposed artificial keratoplasty. Artificial keratoplasty is a surgical method that uses a special optical device made of transparent medical polymer materials to be implanted into the corneal tissue to replace part of the corneal scar tissue and restore vision. Due to unresolved issues such as the rejection reaction of corneal tissue to synthetic materials, the long-term results are not good, and it often causes aqueous humor leakage at the transplant site and graft shedding, so it is not yet widely used. At this stage, artificial corneas are only suitable for blindness after suffering from various serious corneal diseases, especially those who have all corneal leukoplakia caused by severe chemical burns and who have failed multiple corneal transplants and cannot undergo other surgeries.

On the basis of artificial cornea, some researchers have proposed bionic cornea. For example, in the document "Short peptide analogs as alternatives to collagen in pro-regenerative corneal implants", Jangamreddy, JaganmohanR. et al. proposed a PEGylated collagen-like protein, which was chemically cross-linked to obtain a bionic cornea. This bionic cornea has excellent biocompatibility and can effectively solve the problem of corneal tissue's strong rejection reaction to synthetic materials.

However, the strength of this bionic cornea is very low, only 0.022MPa. If this bionic cornea is used for corneal transplantation, there are still problems such as difficulty in transplantation and the possibility of causing corneal keratoconus.

### SUMMARY OF THE INVENTION

In order to solve the problem of low strength of the existing bionic cornea, this application provides a bionic cornea, wherein a raw material of the bionic cornea is a PEGylated collagen-like protein; the PEGylated collagen-like protein includes a collagen-like protein and a polyethylene glycol derivative modified on the collagen-like protein; and the polyethylene glycol derivative include PEG-40k and PEG-20k.

In one embodiment of the present application, the bionic cornea is produced by cross-linking PEGylated collagen-like protein and then curing it.

In one embodiment of the present application, the polyethylene glycol derivative consists of PEG-40k and PEG-20k.

In one embodiment of the present application, the amino acid sequence of the collagen-like protein is as shown in SEQ ID NO.1. In SEQ ID NO.1, X is 4 Hyp (4-hydroxyproline), that is:
The amino acid sequence of SEQ ID NO.1 is:

H-Pro-Lys-Gly-Pro-Lys-Gly-Pro-Lys-Gly-Pro-Lys-Gly-Pro-Hyp-Gly-Pro-Hyp-Gly-Pro-Hyp-Gly-
Pro-Hyp-Gly-Asp-Hyp-Gly-Asp-Hyp-Gly-Asp-Hyp-Gly-Asp-Hyp-Gly-OH

In one embodiment of the present application, the molar ratio of PEG-40k and PEG-20k is 0.5 to 6:1.

In one embodiment of the present application, the molar ratio of PEG-40k and PEG-20k is 2 to 3:1. In one embodiment of the present application, the number of activating groups of the PEG-40k is one or more of 8-arm, 4-arm, 2-arm or 1-arm; the number of activating groups of the PEG-20k is one or more of 8-arm, 4-arm, 2-arm or 1-arm.

In one embodiment of the present application, the number of activating groups of PEG-40k is 4-arm or 8-arm; the number of activating groups of the PEG-20k is 4-arm or 8-arm.

In one embodiment of the present application, the activating group of the PEG-40k is one or more of -MAL, -NHS, -SG, -SPA, -SS or -EDC; the activating group of the PEG-20k is one or more of -MAL, -NHS, -SG, -SPA, -SS or -EDC.

In one embodiment of the present application, the activating group of PEG-40k is -MAL; the activating group of PEG-20k is -MAL.

In one embodiment of the present application, one end of the collagen-like protein is connected to a linker; the polyethylene glycol derivative is modified on the linker through an activating group.

In one embodiment of the present application, the linker is connected to the N-terminus of collagen-like protein.

In one embodiment of the present application, the amino acid sequence of the linker is shown in SEQ ID NO. 2 or SEQ ID NO. 3.

In one embodiment of the present application, the modification site of the polyethylene glycol derivative on the linker is one or more of a thiol group, an amino group, a carboxyl group or an imidazole group.

In one embodiment of the present application, when the amino acid sequence of the linker is as shown in SEQ ID NO. 2, the modification site of the polyethylene glycol derivative on the linker is a thiol group; when the amino acid sequence of the linker is as shown in SEQ ID NO. 3, the modification site of the polyethylene glycol derivative on the linker is an amino group.

In one embodiment of the present application, the core conformation of the PEGylated collagen-like protein is one or more of HG or TP.

In one embodiment of the present application, the core conformation of the polyethylene glycol derivative in the PEGylated collagen-like protein is TP.

In one embodiment of the present application, the amino acid configuration of the collagen-like protein in the PEGylated collagen-like protein is one or more of D-type or L-type.

In one embodiment of the present application, the molecular weight of the PEGylated collagen-like protein is 15,000 to 75,000 Da.

In one embodiment of the present application, the molecular weight of the PEGylated collagen-like protein is 30,000 to 75,000 Da.

This application also provides a method for preparing the above-mentioned bionic cornea, wherein the method includes the following steps: a reaction step: reacting a collagen-like protein with a polyethylene glycol derivative at a pH value of 4.0 to 10.0 and a temperature of 2°C to 40°C for 1 hour to 48 hours to obtain the reaction product;
a freeze-drying step: freeze-drying the reaction product to obtain a freeze-dried preparation;
a cross-linking step: dissolving the freeze-dried preparation in a buffer to obtain a dissolving solution; mixing the dissolving solution with a MPC mother solution to obtain a mixed solution 1; mixing the mixed solution 1 with a DMTMM mother solution to obtain a mixed solution 2; and
a curing step: pouring the mixed solution 2 into a cornea mold and standing to obtain a crude cornea.

In one embodiment of the present application, the reaction step is: reacting the collagen-like protein with the polyethylene glycol derivative at a pH value of 6.0 to 8.0 and a temperature of 2°C to 8°C for 5 hours to 8 hours.

In one embodiment of the present application, in the reaction step, the molar ratio of the collagen-like protein to the polyethylene glycol derivative is 1 to 16:1.

In one embodiment of the present application, in the reaction step, the molar ratio of collagen-like protein to the polyethylene glycol derivative is 8 to 12:1.

In one embodiment of the present application, in the reaction step, a reaction solvent of the collagen-like protein and the polyethylene glycol derivative is water or a dilute hydrochloric acid solution.

In one embodiment of the present application, the concentration of the dilute hydrochloric acid solution is 1 mmol/L to 10mmol/L; the pH value of the dilute hydrochloric acid solution is adjusted to 6.0 to 8.0 with a dilute alkali solution.

In one embodiment of the present application, the dilute alkali solution is a sodium hydroxide solution or ammonia water with a pH value of 9.0 to 11.0.

In one embodiment of the present application, in the reaction step, the feed concentration of the collagen-like protein in the reaction solvent is 1 mg/mL to 15 mg/mL.

In one embodiment of the present application, in the reaction step, the feed concentration of the collagen-like protein in the reaction solvent is 8 mg/mL to 10 mg/mL.

In one embodiment of the present application, the freeze-drying step is: mixing the reaction product and a freeze-drying protective agent and then freeze-drying to obtain a freeze-dried preparation.

In one embodiment of the present application, the freeze-drying protective agent is one or more of mannitol, sucrose or alanine.

In one embodiment of the present application, the freeze-drying includes the following stages:
stage 1: freeze-drying for 6 hours at a temperature of -45°C and a vacuum of 500m Torr;
stage 2: freeze-drying for 17 hours at a temperature of -30°C and a vacuum of 100m Torr; and
stage 3: freeze-drying for 7 hours at a temperature of 25°C and a vacuum of 100m Torr.

In one embodiment of the present application, in the cross-linking step, the pH value of the buffer is 5.5 to 8.0. In one embodiment of the present application, in the cross-linking step, the pH value of the buffer is 6.5 to 7.5. In one embodiment of the present application, in the cross-linking step, the concentration of the buffer is 0.5 mol/L to 0.7mol/L.

In one embodiment of the present application, in the cross-linking step, the buffer is MOPS Buffer, MES Buffer or PBS Buffer.

In one embodiment of the present application, in the cross-linking step, the concentration of the PEGylated collagen-like protein in the buffer is 5 g/mL to 40g/mL.

In one embodiment of the present application, in the cross-linking step, the concentration of the PEGylated collagen-like protein in the buffer is 12 g/mL to 18g/mL.

In one embodiment of the present application, in the cross-linking step, the concentration of the PEGylated collagen-like protein in the buffer is 15g/mL.

In one embodiment of the present application, in the cross-linking step, the mixing mass ratio of the dissolving solution and the MPC mother solution is 2:1 to 4:1.

In one embodiment of the present application, in the cross-linking step, the mixing mass ratio of the mixed solution 1 and the DMTMM mother solution is 5:1 to 7:1.

In one embodiment of the present application, the cross-linking step is completed at a temperature of 25°C to 60°C. In an embodiment of the present application, the cross-linking step is completed at a temperature of 45°C to 55°C.

In one embodiment of the present application, in the curing step, the standing is performed at a temperature of 4°C to 35°C. In one embodiment of the present application, in the curing step, the standing is performed at a temperature of 4°C to 25°C. In one embodiment of the present application, in the curing step, the standing is performed for 8 hours to 20 hours. In one embodiment of the present application, the components of the MPC mother solution include MPC (2-methacryloyloxyethylphosphocholine), PEGDA (polyethylene glycol) diacrylate), TEMED (N, N, N', N'-tetramethylethylenediamine) and a solvent.

In one embodiment of the present application, in the MPC mother solution, the concentration of MPC is 15 g/mL to 40g/mL; in terms of volume percentage, the concentration of PEGDA in the MPC mother solution is 0.6% to 15%, and the concentration of TEMED is 0.05% to 2%.

In one embodiment of the present application, the concentration of PEGDA in the MPC mother solution is 8% to 12%. In one embodiment of the present application, in the MPC mother solution, the concentration of PEGDA is 10%. In one embodiment of the present application, the solvent of the MPC mother solution is a buffer; the pH value of the buffer is 6.0 to 8.0.

In one embodiment of the present application, in the MPC mother solution, the pH value of the buffer is 6.5 to 7.5. In an embodiment of the present application, in the MPC mother solution, the concentration of the buffer is 0.5 mol/L to 0.7mol/L.

In one embodiment of the present application, in the MPC mother solution, the buffer is MOPS Buffer, MES Buffer or PBS Buffer.

In one embodiment of the present application, the components of the DMTMM mother solution include DMTMM (4-(4, 6-dimethoxytriazin-2-yl)-4-methylmorpholine hydrochloride), APS (ammonium persulfate) and a solvent.

In one embodiment of the present application, in the DMTMM mother solution, the concentration of DMTMM is 5 g/mL to 20g/mL, and the concentration of APS is 0.5 g/mL to 5g/mL.

In one embodiment of the present application, the solvent of the DMTMM mother solution is a buffer; the pH value of the buffer is 6.0 to 8.0.

In one embodiment of the present application, in the DMTMM mother solution, the pH value of the buffer is 6.5 to 7.5.

In one embodiment of the present application, in the DMTMM mother solution, the concentration of the buffer is 0.5 mol/L to 0.7mol/L.

In one embodiment of the invention, in the DMTMM mother solution, the buffer is MOPS Buffer, MES Buffer or PBS Buffer.

In one embodiment of the present application, after the curing step, the method further includes a soaking step; the soaking step is: adding the crude cornea together with the mold into the buffer for a first soaking; after the first soaking is completed, opening the mold for a second soaking; after the second soaking is completed, demoulding and obtaining the finished cornea.

In one embodiment of the present application, in the soaking step, the pH value of the buffer is 5.5 to 8.0. In one embodiment of the present application, in the soaking step, the pH value of the buffer is 6.5 to 7.5.

In one embodiment of the present application, in the soaking step, the concentration of the buffer is 0.05 mol/L to 1 mol/L.

In one embodiment of the present application, in the soaking step, the buffer is MOPS Buffer, MES Buffer, or PBS Buffer.

In an embodiment of the present application, the first soaking is performed at a temperature of 4°C to 35°C for 5 hours to 24 hours. In an embodiment of the present application, the second soaking is performed at a temperature of 4°C to 35°C for 3 hours to 10 hours.

In one embodiment of the present application, after the reaction step and before the freeze-drying step, the method further includes a purification step; the purification step is to intercept substances with a molecular weight of greater than or equal to 30,000 Da in the reaction product by filtration to obtain the PEGylated collagen-like protein.

In one embodiment of the present application, the filtration is dialysis or ultrafiltration.

The technical solution of this application has the following advantages:
1. This application provides a bionic cornea. The raw material of the bionic cornea is PEGylated collagen-like protein. The PEGylated collagen-like protein includes collagen-like protein and PEG-40k and PEG-20k modified on the collagen-like protein. The modification of PEG-40k and PEG-20k can significantly improve the cross-linking properties of collagen-like proteins, making the strength of the cornea of this application nearly 20 times higher than that of corneas made of collagen-like proteins. The strength of the cornea made of other PEGylated collagen-like proteins is increased by nearly 12 times (the strength of the cornea made of the PEGylated collagen-like proteins as reported in the article "Short peptide analogs as alternatives to collagen in pro-regenerative corneal implants" is only 0.022MPa). Therefore, the cornea of this application has great application prospects in the field of corneal transplantation.

Further, the molar ratio of PEG-40k and PEG-20k is 2 to 3:1; the PEG modification at this molar ratio has a better effect on improving the cross-linking properties of collagen-like proteins, and can further improve the strength of the cornea of the present application.

Further, the PEG-40k is 8-arm-PEG-40k-MAL, and the PEG-20k is 4-arm-PEG-20k-MAL; the PEG modification under this formula has a better effect on improving the cross-linking properties of collagen-like proteins, and can further improve the strength of the cornea of the present application.

2. This application provides a method for preparing the above-mentioned bionic cornea. The method includes four steps: a reaction step, a freeze-drying step, a cross-linking step and a curing step. The reaction step is that collagen-like protein and PEG derivative are reacted at a pH of 6.0 to 8.0 at a temperature of 2°C to 8°C to prepare PEGylated collagen-like protein; the PEGylated collagen-like protein produced under this reaction step has better cross-linking properties and can further improve the strength of the cornea. In addition, compared with other existing preparation methods for PEGylated collagen-like protein, the reaction time of this reaction step is shorter. It only takes 5 hours to 8 hours to obtain PEGylated collagen-like protein that can be made into a hydrogel to form the cornea (in the literature "Short peptide analogs as alternatives to collagen in pro-regenerative corneal implants", it takes 4 weeks to prepare PEGylated collagen-like protein that can be made into a hydrogel to form the cornea), which helps large-scale industrial production of corneas.

Further, in the reaction step, the molar ratio of the collagen-like protein and the polyethylene glycol derivative is 8 to 12:1; the cross-linking properties of the PEGylated collagen-like protein prepared under this molar ratio is better, and it can further improve the strength of the prepared cornea.

Further, in the reaction step, the feed concentration of collagen-like protein in the reaction solvent is 8 mg/mL to 10 mg/mL; the cross-linking properties of the PEGylated collagen-like protein prepared at this feed concentration is better, which can further improve the strength of the cornea obtained.

Further, in the cross-linking step, the freeze-dried preparation is cross-linked at a pH value of 6.5 to 7.5 and a temperature of 45°C to 55°C to prepare a bionic cornea; the bionic cornea prepared under this cross-linking condition has higher strength..

Further, in the cross-linking step, the concentration of PEGylated collagen-like protein is controlled at 12 g/mL to 18g/mL; the bionic cornea produced at this concentration of PEGylated collagen-like protein has higher strength.

Further, in the cross-linking step, the concentration of PEGDA in the MPC mother solution is controlled at 8% to 12%; the bionic cornea produced under this PEGDA concentration has higher strength.

Further, in the freeze-drying step, the PEGylated collagen-like protein is freeze-dried under the protection of a freeze-drying protective agent; the freeze-drying protective agent can protect the PEGylated collagen-like protein, making the structure of the PEGylated collagen-like protein will not be destroyed during the freeze-drying process, which results in better cross-linking properties of the freeze-dried preparation and further improves the strength of the prepared cornea.

### DETAILED DESCRIPTION

The following examples are provided for a better understanding of the application. The application is not limited to the best embodiments described. These examples do not constitute a limitation on the contents and scope of protection of the application. Any person who derives any product identical or similar to the application under the inspiration of the present application, or produced by combining the features of the application with those of the other prior art, will fall within the scope of protection of the application.

Where no specific experimental procedures or conditions are indicated in the following examples, they can be performed in accordance with the operation or conditions of conventional experimental procedures described in the relevant literature in the field. Where no manufacturer is indicated, the reagents or instruments used in these examples are commercially market-available conventional reagents. In the following examples, the synthesis of collagen-like proteins and the connection between collagen-like protein and linker were performed by Shanghai AmbioPharm. InC. The PEG derivatives in the following examples were purchased from Xiamen Sinopeg Bio-technology Co.

### Example 1: A bionic cornea and its preparation

This example provides a bionic cornea, wherein the bionic cornea is produced by cross-linking the PEGylated collagen-like protein and then curing. The PEGylated collagen-like protein consists of the collagen-like protein with the amino acid sequence as shown in SEQ ID NO. 1, a linker attached to the N-terminus of the collagen-like protein with the amino acid sequence as shown in SEQ ID NO. 2, and 8-arm-PEG-40k-MAL and 4-arm-PEG-20k-MAL modified on the thiol group of the linker, in which the molar ratio of 8-arm-PEG-40k-MAL and 4-arm-PEG-20k-MAL was 2:1, and the nucleus conformation of polyethylene glycol derivatives of PEGylated collagen-like protein was TP, and the molecular weight of PEGylated collagen-like protein is 30,000 to 75,000 Da. The bionic cornea was named as Bionic Cornea 1.

The method of preparing the above-described Bionic Cornea 1 includes the following steps:
Reaction step: Collagen-like protein and polyethylene glycol derivative were dissolved in dilute hydrochloric acid solution at a concentration of 5 mmol/L (the pH value of the dilute hydrochloric acid solution was adjusted to 6.5 using sodium hydroxide solution at pH value of 11.0), so that the molar concentrations of collagen-like protein and polyethylene glycol derivative in the dilute hydrochloric acid solution were 2.4 mmol/L and 0.3 mmol/L, respectively, and the reaction system was obtained. The reaction system was reacted at pH value of 6.5 and at a temperature of 5°C for 12h to obtain the reaction product;
Purification step: the reaction product was ultrafiltrated with ultrafiltration film with a pore size of 30,000 Da at 5°C, and the molecular weight of the reaction product greater than or equal to 30,000 Da was intercepted to obtain the PEGylated collagen-like protein.
Freeze-drying step: mix PEGylated collagen-like protein and mannitol in the ratio of 1:5 by mass to obtain a freeze-drying system; freeze-dry the freeze-drying system to obtain the freeze-dried preparation.
Cross-linking step: dissolve the freeze-dried preparation in MES Buffer at a concentration of 0.5 mol/L, pH 5.5, so that the concentration of PEGylated collagen-like protein in the MES Buffer is 12.5 g/mL, and obtain the dissolving solution; mix the dissolving solution with the MPC mother solution according to the mass ratio of 5:1 and obtain the a mixed solution 1; mix the mixed solution 1 with the DMTMM mother solution according to the mass ratio of 7:1 and obtain the mixed solution 2; the whole crosslinking process is completed at 45°C;
Curing step: pour the mixed solution 2 into the corneal mold and leave it at 25°C for 12h to obtain the crude cornea;
Soaking step: add the crude corneal (with the mold) to the PBS Buffer at a concentration of 0.1 mol/L, pH 0.5, and soak at 4 °C for 24h, open the mold, and continue to soak at 4 °C for 4h, demould the matter, to obtain the Bionic Cornea 1;
Wherein the MPC mother solution's formulation is 30 g/mL MPC, 10% PEGDA (v/v), 1% TEMED (v/v), and 0.5 mol/L solvent, pH 5.5 MOPS Buffer.

The DMTMM mother solution's formulation is 10 g/mL MMTMM, 15 g/mL APS, and solvent is 0.5 mol/L MOPS Buffer, pH 5.5.

The freeze-drying step consisted of the following stages:
Stage I: freeze-drying at -45°C for 6h under a vacuum of 500m Torr.
Stage II: freeze-drying at -30°C with a vacuum of 100m Torr for 17h;
Stage III: freeze-drying at a temperature of 25°C and a vacuum of 100m Torr for 7h.

### Example 2: A bionic cornea and its preparation

This example provides a bionic cornea, wherein the bionic cornea is produced based on the Bionic Cornea 1 of example 1 by replacing the PEG formulation used in example 1 (8-arm-PEG-40k-MAL and 4-arm-PEG-20k-MAL with a molar ratio of 2:1) with:
8-arm-PEG-40k-MAL and 4-arm-PEG-20k-MAL with a molar ratio of 1:0;
8-arm-PEG-40k-MAL and 4-arm-PEG-20k-MAL with a molar ratio of 0:1;
8-arm-PEG-40k-MAL and 4-arm-PEG-20k-MAL with a molar ratio of 6:1;
8-arm-PEG-40k-MAL and 4-arm-PEG-20k-MAL with a molar ratio of 4:1;
8-arm-PEG-40k-MAL and 4-arm-PEG-40k-MALwith a molar ratio of 2:1;
8-arm-PEG-40k-MAL, 4-arm-PEG-20k-MAL and 4-arm-PEG-40k-MAL with a molar ratio of 4:2:1:
   8-arm-PEG-40k-MAL, 4-arm-PEG-40k-MAL and 4-arm-PEG-20k-MAL with a molar ratio of 2:2:1;
   8-arm-PEG-40k-MAL, 4-arm-PEG-40k-MAL and 8-arm-PEG-20k-MAL with a molar ratio of 2:2:1.

The above bionic corneal proteins were sequentially named Bionic Corneas 2 to 9.

The preparation method of the above Bionic Corneas 2 to 9 is the same as that of the Bionic Cornea 1.

### Example 3: A bionic cornea and its preparation

This example provides a bionic cornea, wherein the bionic cornea is produced by crosslinking the PEGylated collagen-like protein and then curing, wherein the PEGylated collagen-like protein consists of the collagen-like protein with the amino acid sequence as shown in SEQ ID NO. 1, a linker attached to the N-terminus of the collagen-like protein with the amino acid sequence as shown in SEQ ID NO. 3, and 8-arm-PEG-40k-MAL and 4-arm-PEG-20k-MAL modified on the amino group of the linker, in which the molar ratio of 8-arm-PEG-40k-MAL and 4-arm-PEG-20k-MAL was 2:1, and the nucleus conformation of polyethylene glycol derivatives of the PEGylated collagen-like protein was TP, and the molecular weight of the PEGylated collagen-like protein is 30,000 to 75,000 Da. The bionic cornea was named as Bionic Cornea 10. The preparation method of the above-described Bionic Cornea 10 is the same as that of the Bionic Cornea 1.

### Example 4: A bionic cornea and its preparation

This example provides a bionic cornea, and the bionic cornea is based on the Bionic Cornea 1 of Example 1, the reaction temperature (5°C) in the reaction step is replaced with: 2°C, 8°C, 25°C, 37°C, respectively.

The above bionic corneas were sequentially named Bionic Corneas 11 to 14.

### Example 5: A bionic cornea and its preparation

This example provides a bionic cornea, wherein the bionic cornea is based on the Bionic Cornea 1 of Example 1, wherein the reaction pH (6.5) in the reaction step is replaced with: pH 2.5 , pH 4.5 , pH 6.0 , pH 6.5 , pH 7.0 , pH 8.0 , pH 8.5 , pH 10.5 respectively.

The above bionic corneas were sequentially named Bionic Corneas 15 to 22.

### Example 6: A bionic cornea and its preparation

This example provides a bionic cornea, and the bionic cornea is based on the Bionic Cornea 1 of Example 1, with the molar concentrations of the collagen-like protein and the polyethylene glycol derivative in the dilute hydrochloric acid solution in the reaction step being replaced, respectively:
Let the molar concentrations of collagen-like protein and polyethylene glycol derivatives in dilute hydrochloric acid solution were 3.0 mmol/L and 0.375 mmol/L, respectively.
Let the molar concentrations of collagen-like protein and polyethylene glycol derivatives in dilute hydrochloric acid solution were 1.8 mmol/L and 0.225 mmol/L, respectively;
Let the molar concentrations of collagen-like protein and polyethylene glycol derivatives in dilute hydrochloric acid solution were 1.2 mmol/L and 0.15 mmol/L, respectively.
The above bionic corneas were sequentially named Bionic Corneas 23 to 25.

### Example 7: A bionic cornea and its preparation

This example provides a bionic cornea, and the bionic cornea is produced based on the Bionic Cornea 1 of Example 1, and the reaction time (12h) in the reaction step is replaced with: 4h, 8h, 16h, 24h, respectively.

The above bionic corneas were sequentially named Bionic Corneas 26 to 29.

### Example 8: A bionic cornea and its preparation

This example provides a bionic cornea, the bionic cornea is based on the Bionic Cornea 1 of Example 1, and the freeze-drying protective agent (mannitol) in the freeze-drying step is replaced with: sucrose, alanine, respectively.

The above bionic corneas were sequentially named Bionic Corneas 30 to 31.

### Example 9: A bionic cornea and its preparation

This example provides a bionic cornea, and the bionic cornea is produced based on the Bionic Cornea 1 of Example 1, and the cross-linking temperature (45° C) in the cross-linking step is replaced with: 20° C, 25° C, 40° C, 50° C, 55° C, 60° C, 65° C, respectively.

The above bionic cornea was sequentially named Bionic Corneas 32 to 38.

### Example 10: A bionic cornea and its preparation

This example provides a bionic cornea, and the bionic cornea is produced based on the Bionic Cornea 1 of Example 1, and the pH (5.5) of the Buffer in the cross-linking step is replaced with: 5.0, 6.0, 6.5, 7.0, 7.5, 8.0, 8.5, 9.0, respectively.

The above bionic corneas were sequentially named as Bionic Corneas 39 to 46.

### Example 11: A bionic cornea and its preparation

This example provides a bionic cornea, the bionic cornea being based on the Bionic Cornea 1 of Example 1, and the concentration of PEGylated collagen-like protein in MES Buffer (15g/mL) in the cross-linking step were replaced with 1 g/mL, 5 g/mL, 10 g/mL, 20 g/mL, 25 g/mL, 30 g/mL, 35 g/mL, 40 g/mL, and 45g/mL, respectively.

The above bionic corneas were sequentially named as Bionic Corneas 47 to 55.

### Example 12: A bionic cornea and its preparation

This example provides a bionic cornea, and the bionic cornea is based on the Bionic Cornea 1 of Example 1, and the concentration of PEGDA in the MPC other solution in the cross-linking step (10%, v/v) is replaced with 0.5%, 0.6%, 1%, 5%, 8%, 12%, 15% (v/v), respectively.

The above bionic corneas were sequentially named Bionic Corneas 56 to 62.

### Experimental Example 1: Experiment on the effect of PEG formulation and preparation process on the strength of bionic cornea

This experimental example provides an experiment on the effect of PEG formulation and preparation process on the strength of a bionic cornea, and the experimental procedure is as follows:
Referring to the preparation method of Bionic Cornea 1, the collagen-like protein with the amino acid sequence as shown in SEQ ID NO.1 was directly prepared into bionic corneas, and the bionic corneas were used as a blank control. The strengths of corneas 1 to 62 were tested by using a universal tensile testing machine, and the results of the test are shown in Table 1.

From Table 1, it can be seen that the combination of PEG modification formulation and the preparation process can affect the strength of the bionic cornea; among them, the combination of PEG modification formulation significantly affects the strength and elasticity of the cornea; the reaction temperature and pH have a direct correlation with the conjugation rate between collagen-like proteins and PEG derivatives. They can affect the formation of the polymer network structure, which is a key factor for whether the modification products can be made into hydrogel-formed cornea. The composition of the freeze-drying excipients provides backbone support and protection for the PEGylated collagen-like protein, which affects the spatial conformation and bioactivity of the polymer, resulting in differences in film hardness; the extremely low concentration of PEGylated collagen-like protein results in poor corneal strength, while the extremely high concentration results in a very short corneal curing time, making it impossible to complete the subsequent casting steps; due to the neutral isoelectric point of PEGylated collagen-like protein, when the cross-linking pH is far away from the isoelectric point, the groups involved in the reaction can not be close to each other, which will lead to the phenomenon that the corneal strength will be low or the PEGylated collagen-like protein failed to be the hydrogel form. The hydrogel made of PEGylated collagen-like protein is a temperature-sensitive hydrogel, and its solubility is not good at low temperatures, which will lead to low solid concentrations of corneal and low strength, and the cross-linking speed will be extremely fast at high temperatures, thus making it impossible to pouring the cornea to mold and forming a premature hydrogel; if the concentration of PEGDA in the MPC mother solution is too low, it will lead to deterioration of corneal strength; and if the concentration is too high, it will lead to excessive cross-linking of PEGylated collagen-like protein, which will cause the increase of the brittleness of hydrogel made of PEGylated collagen-like protein and the decrease of the toughness of hydrogel, and then further cause the cornea to deteriorate. In Table 1, corneas 1 to 13, 16 to 21, 23 to 31, 33 to 37, 39 to 44, 49 to 53, and 56 to 62 all have good strengths and good bio-compatibility. They are very promising for application in the field of corneal transplantation.

**Table 1 Strength of Corneas 1 to 62**

| | Group | Strength (MPa) | | Group | Strength (MPa) |
|---|---|---|---|---|---|
| Cornea 1 | | 0.255 | Cornea 33 | | 0.098 |
| Cornea 2 | | 0.083 | Cornea 34 | | 0.211 |
| Cornea 3 | | 0.074 | Cornea 35 | | 0.261 |
| Cornea 4 | | 0.138 | Cornea 36 | | 0.199 |
| Cornea 5 | | 0.201 | Cornea 37 | | 0.160 |
| Cornea 6 | | 0.088 | Cornea 38 | | /(No film formation) |
| Cornea 7 | | 0.173 | Cornea 39 | | 0.051 |
| Cornea 8 | | 0.211 | Cornea 40 | | 0.261 |
| Cornea 9 | | 0.197 | Cornea 41 | | 0.286 |
| Cornea 10 | | 0.204 | Cornea 42 | | 0.270 |
| Cornea 11 | | 0.253 | Cornea 43 | | 0.265 |
| Cornea 12 | | 0.245 | Cornea 44 | | 0.256 |
| Cornea 13 | | 0.123 | Cornea 45 | | /(No film formation) |
| Cornea 14 | | /(No film formation) | Cornea 46 | | /(No film formation) |
| Cornea 15 | | /(No film formation) | Cornea 47 | | /(No film formation) |
| Cornea 16 | | 0.027 | Cornea 48 | | 0.013 |
| Cornea 17 | | 0.241 | Cornea 49 | | 0.136 |
| Cornea 18 | | 0.245 | Cornea 50 | | 0.239 |
| Cornea 19 | | 0.243 | Cornea 51 | | 0.221 |
| Cornea 20 | | 0.234 | Cornea 52 | | 0.189 |
| Cornea 21 | | 0.163 | Cornea 53 | | 0.171 |
| Cornea 22 | | /(No film formation) | Cornea 54 | | /(No film formation) |
| Cornea 23 | | 0.246 | Cornea 55 | | /(No film formation) |
| Cornea 24 | | 0.224 | Cornea 56 | | 0.033 |
| Cornea 25 | | 0.152 | Cornea 57 | | 0.045 |
| Cornea 26 | | 0.116 | Cornea 58 | | 0.117 |
| Cornea 27 | | 0.237 | Cornea 59 | | 0.156 |
| Cornea 28 | | 0.257 | Cornea 60 | | 0.215 |
| Cornea 29 | | 0.253 | Cornea 61 | | 0.219 |
| Cornea 30 | | 0.234 | Cornea 62 | | 0.225 |
| Cornea 31 | | 0.180 | Cornea (blank control) | | 0.014 |
| Cornea 32 | | /(No film formation) | | | |

Obviously, the above-mentioned embodiments are only examples for clear explanation and are not intended to limit the implementation. For those of ordinary skill in the art, other different forms of changes or modifications can be made based on the above description. An exhaustive list of all implementations is neither necessary nor possible. The obvious changes or modifications derived therefrom are still within the scope of protection created by this application.

## Claims

1. A bionic cornea, wherein a raw material of the bionic cornea is a PEGylated collagen-like protein; the PEGylated collagen-like protein comprises a collagen-like protein and a polyethylene glycol derivative modified on the collagen-like protein; and the polyethylene glycol derivative comprises PEG-40k and PEG-20k.

2. The bionic cornea of claim 1, wherein the bionic cornea is produced by cross-linking the PEGylated collagen-like protein and then curing it.

3. The bionic cornea of claim 1 or 2, wherein the amino acid sequence of the collagen-like protein is as shown in SEQ ID NO. 1.

4. The bionic cornea of any one of claims 1 to 3, wherein the molar ratio of the PEG-40k to the PEG-20k is 0.5 to 6: 1.

5. The bionic cornea of claim 4, wherein the molar ratio of the PEG-40k to the PEG-20k is 2 to 3:1.

6. The bionic cornea of any one of claims 1 to 3, wherein the number of activating groups of the PEG-40k is one or more of 8-arm, 4-arm, 2-arm or 1-arm; and the number of activating groups of the PEG-20k is one or more of 8-arm, 4-arm, 2-arm or 1-arm.

7. The bionic cornea of claim 6, wherein the number of activating groups of the PEG-40k is 4-arm or 8-arm; and the number of activating groups of the PEG-20k is 4-arm or 8-arm.

8. The bionic cornea of any one of claims 1 to 3, wherein the activating group of the PEG-40k is one or more of -MAL, -NHS, -SG, -SPA, -SS or -EDC; and the activating group of the PEG-20k is one or more of -MAL, -NHS, -SG, -SPA, -SS or -EDC.

9. The bionic cornea of claim 8, wherein the activating group of the PEG-40k is -MAL; and the activating group of the PEG-20k is -MAL.

10. The bionic cornea of any one of claims 1 to 3, wherein one end of the collagen-like protein is connected to a linker; the said polyethylene glycol derivative is modified on the linker through an activating group.

11. The bionic cornea of claim 10, wherein the linker is connected to the N-terminus of the collagen-like protein.

12. The bionic cornea of any one of claims 1 to 3, wherein the amino acid sequence of the linker is as shown in SEQ ID NO.2 or SEQ ID NO.3.

13. The bionic cornea of claim 12, wherein the modification site of the polyethylene glycol derivative on the linker is one or more of a thiol group, an amino group, a carboxyl group or an imidazole group.

14. The bionic cornea of claim 13, wherein when the amino acid sequence of the linker is as shown in SEQ ID NO.2, the modification site of the polyethylene glycol derivative on the linker is a thiol group; and when the amino acid sequence of the linker is as shown in SEQ ID NO.3, the modification site of the polyethylene glycol derivative on the linker is an amino group.

15. A method for preparing the bionic cornea of any one of claims 1 to 14, wherein the method comprises the following steps:
a reaction step: reacting a collagen-like protein with a polyethylene glycol derivative at a pH value of 4.0 to 10.0 and a temperature of 2°C to 40°C for 1 hour to 48 hours to obtain a reaction product;
a freeze-drying step: freeze-drying the reaction product to obtain a freeze-dried preparation;
a cross-linking step: dissolving the freeze-dried preparation in a buffer to obtain a dissolving solution; mixing the dissolving solution with a MPC mother solution to obtain a mixed solution 1;
and mixing the mixed solution 1 with a DMTMM mother solution to obtain a mixed solution 2; and
a curing step: pouring the mixed solution 2 into a corneal mold and standing to obtain a crude corneal.

16. The method of claim 15, wherein the reaction step is: reacting the collagen-like protein with the polyethylene glycol derivative at pH value of 6.0 to 8.0 and a temperature of 2°C to 8°C for 5 hours to 8 hours.

17. The method of claim 15 or 16,wherein in the reaction step, the molar ratio of the collagen-like protein to the polyethylene glycol derivative is 1 to 16:1.

18. The method of claim 17, wherein in the reaction step, the feeding molar ratio of the collagen-like protein to the polyethylene glycol derivative is 8 to 12:1.

19. The method of any one of claims 15 to 18, wherein in the reaction step, a reaction solvent for the collagen-like protein and the polyethylene glycol derivative is water or a dilute hydrochloric acid solution.

20. The method of claim 19, wherein the concentration of the the dilute hydrochloric acid solution is 1 mmol/L to 10 mmol/L; the pH value of the dilute hydrochloric acid solution is adjusted to 6.0 to 8.0 with a dilute alkaline solution.

21. The method of any one of claims 15 to 18, wherein in the reaction step, the feed concentration of collagen-like protein in the reaction solvent is 1 mg/mL to 15 mg/mL.

22. The method of claim 21, wherein in the reaction step, the feed concentration of collagen-like protein in the reaction solvent is 8 mg/mL to 10 mg/mL.

23. The method of any one of claims 15 to 18, wherein the freeze-drying step is: mixing the reaction product and a freeze-drying protective agent, and then freeze-drying to obtain a freeze-dried preparation.

24. The method of claim 23, wherein the freeze-drying protective agent is one or more of mannitol, sucrose or alanine.

25. The method of any one of claims 15 to 18, wherein in the cross-linking step, the pH value of the buffer is 5.5 to 8.0.

26. The method of claim 25, wherein in the cross-linking step, the pH value of the buffer is 6.5 to 7.5.

27. The method of any one of claims 15 to 18, wherein in the cross-linking step, the concentration of the PEGylated collagen-like protein in the buffer is 5 g/mL to 40 g/mL.

28. The method of claim 27, wherein in the cross-linking step, the concentration of the PEGylated collagen-like protein in the buffer is 12 g/mL to 18 g/mL.

29. The method of any one of claims 15 to 18, wherein in the cross-linking step, the mixing mass ratio of the dissolving solution and the MPC mother solution is 2:1 to 4:1.

30. The method of any one of claims 15 to 18, wherein in the cross-linking step, the mixing mass ratio of the mixed solution 1 and the DMTMM mother solution is 5:1 to 7:1.

31. The method of any one of claims 15 to 18, wherein the cross-linking step is completed at a temperature of 25°C to 60°C.

32. The method of claim 31, wherein the cross-linking step is completed at a temperature of 45°C to 55°C.

33. The method of any one of claims 15 to 18, wherein in the curing step, the standing is performed at a temperature of 4°C to 35°C.

34. The method of any one of claims 15 to 18, wherein the component of the MPC mother solution comprises MPC, PEGDA, TEMED and a solvent.

35. The method of claim 34, wherein in the MPC mother solution, the concentration of MPC is 15 g/mL to 40g/mL; in the MPC mother solution, the concentration of PEGDA is 0.6% to 15% and the concentration of TEMED is 0.05% to 2% in terms of volume percentage.

36. The method of claim 35, wherein the concentration of PEGDA in the MPC mother solution is 8% to 12%.

37. The method of any one of claims 15 to 18, wherein the solvent of the MPC mother solution is a buffer; the pH value of the buffer is 6.0 to 8.0.

38. The method of claim 37, wherein the component of the DMTMM mother solution comprises DMTMM, APS and a solvent.

39. The method of claim 38, wherein the concentration of DMTMM in the DMTMM mother solution is 5 g/mL to 20 g/mL, and the concentration of APS is 0.5 g/mL to 5 g/mL.

40. The method of any one of claims 15 to 18, wherein the solvent of the DMTMM mother solution is a buffer; the pH value of the buffer is 6.0 to 8.0.

41. The method of any one of claims 15 to 18, wherein after the curing step, the method further comprises a soaking step; the soaking step is: adding the crude cornea together with the mold into the buffer for a first soaking, after the first soaking is finished, opening the mold for a second soaking, after the second soaking is finished, demoulding, and obtaining a finished cornea.

42. The method of claim 41, wherein in the soaking step, the pH value of the buffer is 5.5 to 8.0.

43. The method of any one of claims 41 to 42, wherein the first soaking is performed at a temperature of 4°C to 35°C for 5 hours to24 hours.

44. The method of any one of claims 41 to 43, wherein the second soaking is performed at a temperature of 4°C to 35°C for 3 hours to 10 hours.

45. The method of any one of claims 15 to 18, wherein after the reaction step and before the freeze-drying step, the method further comprises a purification step; the purification step is to intercept substances with a molecular weight of greater than or equal to 30,000 Da in the reaction product by filtration to obtain the PEGylated collagen-like protein.
